# EUROPEAN PATENT SPECIFICATION

(11) **EP 1 302 182 B1**
(45) Date of publication and mention of the grant of the patent: **20.08.2008**
(21) Application number: 02027647.3
(22) Date of filing: 06.06.1996
(51) Int. Cl.: A61F 2/44

(54) **Translateral spinal implant**
Translaterales Wirbelsäulenimplantat
Implant translatéral du rachis

(30) Priority: 07.06.1995 US 479596
(43) Date of publication of application: 16.04.2003
(62) Divisional of application: 96917995.1
(73) Proprietor: Warsaw Orthopedic, Inc., Warsaw, IN 46581 (US)
(72) Inventor: Michelson, Gary Karlin, Venice, CA 90291 (US)
(74) Representative: Grünecker, Kinkeldey, Stockmair & Schwanhäusser Anwaltssozietät

(56) References cited:
- EP-A- 0 637 440
- WO-A-95/26164
- US-A- 5 062 845

## Description

### BACKGROUND OF THE INVENTION

### Related Applications

This is a continuation in part of copending Application Serial No. 08/074,781 entitled THREADED SPINAL IMPLANT filed on June 10, 1993, which is a continuation in part of Application Serial No. 07/698,674 filed on May 10, 1991 which is a divisional of Application Serial No. 07/205,935 filed on June 13, 1988, now United States Patent No. 5,015,247.

This application is also continuation in part of copending Application Serial No. 08/263,952 entitled ARTIFICIAL SPINAL FUSION IMPLANTS filed on June 22, 1994, which is a continuation of Application Serial No. 08/052,211 filed on April 22, 1993, which is a continuation of Application Serial No. 07/546,849, filed on July 2, 1990, which is a continuation of Application Serial No. 07/212,480 filed on June 28, 1988.

This application is also a continuation in part of Application Serial No. 08/390,131 entitled IMPROVED INTERBODY SPINAL FUSION IMPLANTS filed on February 17, 1995.

This application is also a continuation in part of Application Serial No. 08/394,836 entitled IMPROVED METHODS AND INSTRUMENTATION FOR THE SURGICAL CORRECTION OF HUMAN THORACIC AND LUMBAR SPINAL DISEASE FROM THE LATERAL ASPECT OF THE SPINE, filed on February 27, 1995.

### Field of the Invention

This invention relates generally to spinal fusion implants, and more particularly to spinal fusion implants for insertion from the side of a patient (translateral) across the transverse width of the spine and between two adjacent vertebrae.

### Description of the Related Art

In the past, spinal fusion implants have been inserted only from either an anterior or posterior direction, from the front or the back of the patient. Such implants are well known in the art and may have cylindrical, rectangular and other shapes. In the past, Cloward, Wilterberger, Crock, Viche, Bagby, Brantigan, and others have taught various methods involving the drilling of holes across the disc space between two adjacent vertebrae of the spine for the purpose of causing an interbody spinal fusion. Cloward taught placing a dowel of bone within that drilled hole for the purpose of bridging the defect and to be incorporated into the fusion. Viche taught the threading of that bone dowel. Bagby taught the placing of the bone graft into a metal bucket otherwise smooth on its surface, except for rows of radially placed holes communicative to the interior of the basket and to the bone graft. The Bagby device was disclosed as capable of being used in a horse. Brantigan taught the use of inert blocks preferably made of metal and having that metal at its external surface imitate the porosity of bone. Brantigan theorized that the bone dowel could be replaced entirely with a metal plug that, while not itself active in the fusion, would nevertheless serve to support the vertebrae from within the disc space while allowing fusion to occur around it.

U.S. Patent No. 3,844,601 issued to Ma et al. on November 19, 1974, teaches a method and instrumentation for preparing rectangular spaces across the disc space into the adjacent vertebrae and for preparing a rectangular graft of the bone itself that is inserted in the rectangular spaces.

U.S. Patent No. 4,743,256 issued to Brantigan on May 10, 1988 teaches the use of an inert artificial spacer in the shape of a rectangle in place of using a rectangular bone graft as taught by Ma et al.

U.S. Patent No. 4,878,915 issued to Brantigan on November 7, 1989, teaches the use of fully cylindrical inert implants for use in interbody spinal fusion. Such implants do not participate in the bone fusion process but act as inert spacers and allow for the growth of bone to the outer surfaces of the implants.

U.S. Patent No. 4,834,757 issued to Brantigan on May 30, 1989, teaches a rectangular shaped, hollow spinal fusion implant for use in lieu of a rectangular bone graft or Brantigan's earlier artificial inert spacer.

However, all of the prior implants have been inserted from either the front or the back of the patient. As a result, the spinal fusion implants of the past were necessarily limited in size to the dimensions of the vertebrae relative to the direction in which the implants were inserted. For example, the maximum possible length for an implant that is inserted from either the front or the back of the patient is limited to the depth of the vertebrae, the depth of a vertebrae being the dimension of the vertebrae measured from the anterior end to the posterior end of the vertebrae. It was not previously possible to insert an implant that had a length that was greater than the depth of the vertebrae from front to back as such an implant would protrude from either the anterior or posterior aspect of the spine resulting in great harm to the patient.

In U.S. Patent No. 5,015,247 to Michelson, a cylindrical threaded implant is described for insertion across the disc space between two adjacent vertebrae. Such an implant was disclosed as being inserted either from the front of the patient or from the back and has a diameter larger than the disc space so that it engages both of the adjacent vertebrae.

The maximum diameter possible with a cylindrical implant that is inserted from the front or the back of the patient is limited by at least two factors. The first factor limiting the diameter of a cylindrical implant is realized when an attempt is made to use a single, centrally placed implant from either the front or the back of the patient. Such an implant must be large enough to occupy a sufficient portion of the transverse width of the disc space to promote firm stability. The use of an implant that is placed in the disc space to stabilize the two adjacent vertebrae requires that the vertebrae be stable when the implant is in place, otherwise there will not be any bone bridging between the implant and the vertebrae. If a single implant is used in the center of the disc space, inherent instability is created, as the vertebrae are generally free to rock back and forth over the implant which serves as a fulcrum. However, to achieve the required stability, it would be necessary to use the widest possible implant and the excursion of such a large single implant into the adjacent vertebrae would be so severe that the two vertebrae would be virtually cut in half.

The second factor which limits the diameter size of a cylindrical implant is in the situation where two cylindrical implants are implanted from either the front or the back of the patient and placed side-by-side across a disc space and into the two adjacent vertebrae in an attempt to gain stability while avoiding the problems of the single implant. Such implants require a diameter that is sufficiently large to penetrate into and significantly engage each of the adjacent vertebrae yet the diameter may not be so large that it is no longer possible to place two such implants side-by-side and to still have them contained within the transverse width of the spine.

The use of multiple implants requires that the implants be small enough so as to fit into the same limited spinal width. These implants being of smaller diameter as limited by the need to place more than one within the width of the spine then penetrate only minimally into the depth of the vertebral bone.

Also, the insertion of multiple implants requires multiple procedures, essentially a duplication of any procedure done on one side of the center line must also be performed on the other side of the center line.

Therefore, there exists a need for a spinal fusion implant that is inserted from the translateral approach to the spine that is capable of stabilizing the vertebrae adjacent to such an implant in order to permit bone bridging between the vertebrae and the implant to ultimately achieve fusion of the adjacent vertebrae.

### SUMMARY OF THE INVENTION

The present invention discloses a spinal fusion implant that is inserted from the side of the patient, herein referred to as the translateral approach to the spine. The translateral spinal fusion implant of the present invention is inserted into the spine of a patient across the transverse width of the vertebrae to be fused. The transverse width of a vertebra is measured from one lateral aspect of the spine to the opposite lateral aspect. The depth of a vertebra is measured from the anterior aspect to the posterior aspect of the spine.

As the translateral spinal fusion implant of the present invention is inserted substantially along the transverse width of the vertebrae or at a slight angle to the vertebrae, it has a different structural configuration as compared to spinal implants for insertion from either the front or the back of the patient, as such implants are necessarily limited by the depth, measured from the back of the vertebrae.

In one embodiment of the translateral spinal fusion implant of the present invention, the implant is dimensioned to fit within a bore created across the disc space and into the adjacent vertebrae. Such an implant has a mid-longitudinal axis and opposed arcuate portions for engaging each of the adjacent vertebral bodies, said implant having a maximum cross sectional dimension transverse to the mid-longitudinal axis in the range of 22 mm to 30 mm.

A number of materials are possible for a spinal implant and the implant may comprise surface roughenings and may also have openings capable of retaining fusion promoting material.

According to an advantageous embodiment of the invention, the implant is modular wherein it may comprise a plurality of modular members.

The opposed arcuate portions may be contoured so as to conform to the shape of the disk space and the adjacent vertebral endplate surfaces. One possibility is a substantially cylindrical configuration for the opposed arcuate portions.

The implant may comprise at least in part fusion promoting and/or bioactive materials for active participation of the implant in the spinal fusion process. The implant may also comprise an osteogenic material.

Further advantageous embodiments of the invention are disclosed by the subclaims.

### OBJECT OF THE PRESENT INVENTION

It is an object of the present invention to provide a spinal fusion implant that is easy to insert into the spine and simultaneously provide a good stability of the vertebrae being fused and that is less likely to fail.

This and other objects of the present invention will become apparent from review of the accompanying drawings and the detailed description of the drawings.

### BRIEF DESCRIPTION OF THE DRAWINGS

Figure 1 is a perspective side view of the translateral spinal fusion implant of the present invention having an external thread for engaging the bone of two adjacent vertebrae.

Figure 2 is an elevational view of the anterior aspect of a segment of the spinal column with the spinal fusion implant of Figure 1 inserted from the lateral aspect along the transverse width of the vertebrae.

Figure 3 is an elevational view of the lateral aspect of a segment of the lumbar spine with a first spinal fusion implant of the present invention inserted from the lateral aspect into a hole drilled across a first disc space and into two adjacent vertebrae, and a second spinal fusion implant of the present invention inserted from the lateral aspect into a second hole drilled across a second disc space and into two adjacent vertebrae.

Figure 4 is top sectional view along lines 4--4 of Figure 3 showing the area of contact of the spinal fusion implant of the present invention and the vertebra.

Figure 5 is an anterior elevational view of a segment of the lumbar spine with two cylindrical implants which do not form a part of the invention inserted from the anterior of the spine into holes drilled across the same disc space and into two adjacent vertebrae.

Figure 6 is sectional view along lines 6--6 of Figure 5 showing the area of contact between the two implants of Figure 5 and the vertebra.

Figure 7 is a anterior perspective view of a single vertebra and an alternative embodiment of the spinal fusion implant of the present invention in the form of a dowel inserted translaterally into a hole drilled across a disc space and into the vertebra along the transverse width of the vertebra.

Figure 8 is a perspective view of an alternative embodiment of the spinal fusion implant of the present invention having ratchetings for engaging the vertebrae.

Figure 9 is a perspective view of an alternative embodiment of the spinal fusion implant of the present invention having a knurled surface for engaging the vertebrae.

Figure 10 is a perspective view of an alternative embodiment of the spinal fusion implant of the present invention having ratchetings for engaging the vertebrae and a flattened side.

Figure 11 is a perspective view of an alternative embodiment of the spinal fusion implant of the present invention having a knurled surface for engaging the vertebrae and a flattened side.

Figure 12 is a perspective view of an alternative embodiment of the spinal fusion implant of the present invention having a blasted surface for engaging the vertebrae.

Figure 13 is a perspective view of an alternative embodiment of the spinal fusion implant of the present invention having ratchetings for engaging the vertebrae with openings in the form of vertical and horizontal slots.

Figure 14 is a perspective view of an alternative embodiment of the spinal fusion implant of the present invention having longitudinal splines for engaging the vertebrae and openings in the form of vertical slots.

Figure 15 is elevational view of the lateral aspect of the spinal column having the spinal fusion implant of Figure 14 inserted from the lateral aspect along the transverse width of the vertebrae into a hole created across the disc space and into two adjacent vertebrae.

Figure 16 is a perspective side view of a spinal fusion implant which does not form a part of the invention.

Figure 17 is a elevational anterior view of a segment of the spinal column having the spinal fusion implant of Figure 16 inserted from the lateral aspect in the disc space between two adjacent vertebrae along the transverse width of the vertebrae.

Figure 18 is a perspective side view of a spinal fusion implant which does not form a part of the invention.

Figure 19 is a perspective lateral anterior view of a segment of the spinal column with a plurality of the spinal implants of Figure 18 shown in hidden line inserted from the lateral aspect in a modular fashion in the disc space between two adjacent vertebrae along the transverse width of the vertebrae.

Figure 20 is perspective view of a spinal fusion implant which does not form a part of the invention.

### DETAILED DESCRIPTION OF THE DRAWINGS

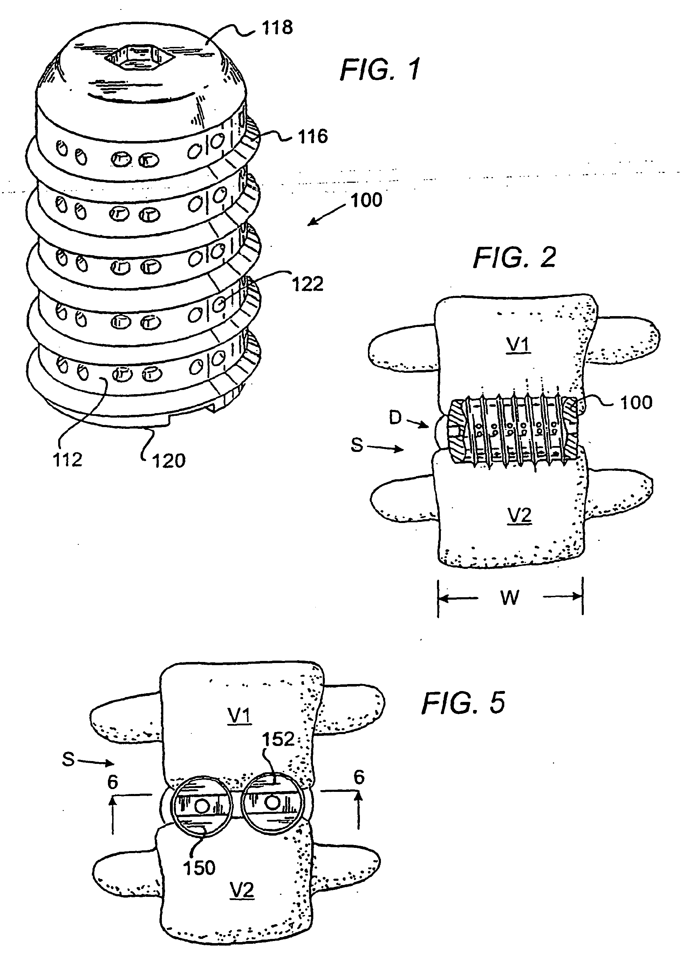

Referring to Figures 1-5, an embodiment of the translateral spinal fusion implant of the present invention, generally referred to by numeral 100, is shown. The spinal fusion implant 100 has a substantially cylindrical configuration having an outer wall 112 surrounding an internal chamber 114 for holding fusion promoting material. The exterior of the spinal fusion implant 100 comprises an external thread 116 suitable for engaging the vertebrae of the spine to stabilize the spinal fusion implant 100 across the disc space and into adjacent vertebrae once surgically implanted. The spinal fusion implant 100 has a removable cap 118 at one end which provides access to the internal chamber 114 and has an insertion end 120 adapted to engage insertion instrumentation.

The cap 118 is removable to provide access to the internal chamber 114, such that the internal chamber 114 can be filled and hold any natural or artificial osteoconductive, osteoinductive, osteogenic, or other fusion enhancing material. Some examples of such materials are bone harvested from the patient, or bone growth inducing material such as, but not limited to, hydroxyapatite, hydroxyapatite tricalcium phosphate; or bone morphogenic protein. The cap 118 and/or the spinal fusion implant 100 itself is made of material appropriate for human implantation such as titanium and/or may be made of, and/or filled and/or coated with a bone ingrowth inducing material such as, but not limited to, hydroxyapatite or hydroxyapatite tricalcium phosphate or any other osteoconductive, osteoinductive, osteogenic, or other fusion enhancing material.

The outer wall 112 comprises openings 122 which may be closed wells or openings communicating into the internal chamber 114 to permit bone ingrowth into the chamber 114.

Referring specifically to Figure 2, an elevational view of the anterior aspect of a segment of the spinal column S with the spinal fusion implant 100 inserted from the lateral aspect of the spinal column S into a hole bored into the adjacent vertebrae V₁ and V₂ across the disc space D. The spinal fusion implant 100 is inserted along the transverse width W of the adjacent vertebrae V₁ and V₂ such that the spinal fusion implant 100 extends translaterally in the direction from one lateral aspect of the vertebrae to the opposite lateral aspect of the vertebrae.

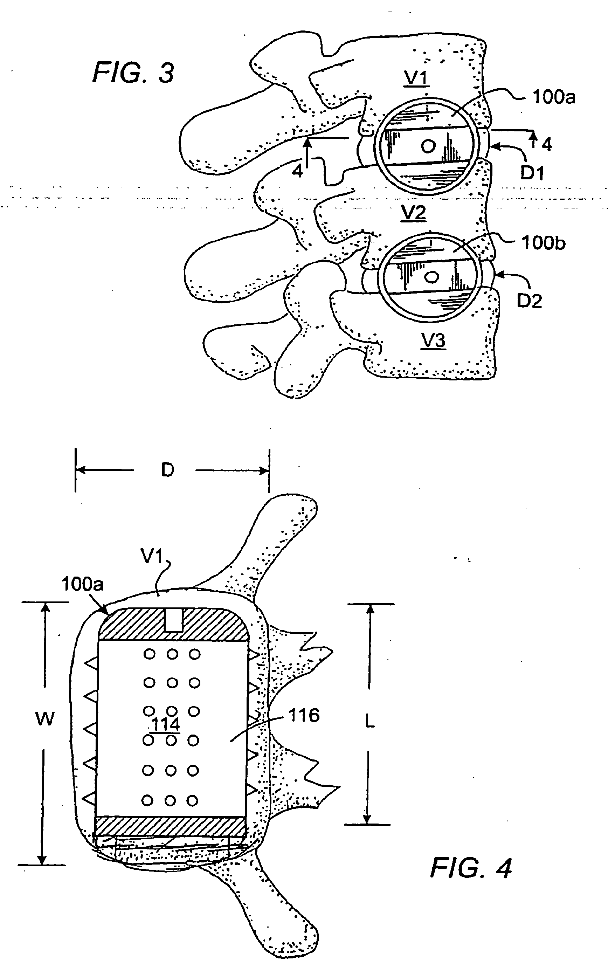

Referring to Figure 3, an elevational view of the lateral aspect of a segment of the lumbar spine S is shown with a first implant 100a, identical to spinal fusion implant 100, inserted from the lateral aspect into a hole bored across a first disc space D₁ and into two adjacent vertebrae V₁ and V₂, and a second implant 100b, identical to spinal fusion implant 100, inserted from the lateral aspect into a second hole bored across a second disc space D₂ and into two adjacent vertebrae V₂ and V₃.

The translateral implants of the present invention are inserted by the translateral method disclosed in copending Application Serial No. 08/394,836 entitled IMPROVED METHODS AND INSTRUMENTATION FOR THE SURGICAL CORRECTION OF HUMAN THORACIC AND LUMBAR SPINAL DISEASE FROM THE LATERAL ASPECT OF THE SPINE, filed on February 27, 1995, which is incorporated herein by reference.

Referring to Figure 4, a top sectional view along lines 4--4 of Figure 3 is shown illustrating the area of contact of the implant 100a and the vertebra V₁. The vertebra V₁ has a depth D measured from the anterior to posterior aspect of the spine, and a transverse width W measured from one lateral aspect to the opposite lateral aspect of the vertebra V₁. The implant 100a has a length L that is substantially greater than the depth D of the vertebra
V₁, such that the implant 100a may extend substantially across the transverse width W of the vertebra V₁. In the preferred embodiment, the implant 100a has a length L that is greater than one half the transverse width W of the vertebrae and has a diameter of a sufficiently large size that approximates the depth D of the vertebra V₁. As a result of the large length and diameter of the implant 100a, a large surface area of contact between the implant 100a and the vertebrae V₁ is possible creating a highly stable construct. The implant 100a has a much greater surface area of contact with the vertebra V₁ than was previously possible with implants that are inserted from the front or the back of the spine.

As described above in the Background of the Invention, a centrally placed single implant from either the front or the back of the patient must be large enough to occupy a sufficient portion of the transverse width W of the vertebrae to promote firm stability. However, the vertical height of such an implant and excursion into the adjacent vertebrae would be so severe that if any two consecutive disc spaces were to be operated upon, the vertebra in between the disc spaces would be cut in half. Therefore it has been the practice to use multiple implants, one on each side of the center line (mid-saggital axis) of the vertebrae, thereby providing a greater degree of stability. Referring to Figure 5, an anterior elevational view of a segment of the lumbar spine S is shown with two cylindrical implants 150 and 152 inserted from the anterior aspect of the spine S into holes drilled across the same disc space D and into two adjacent vertebrae V₁ and V₂.

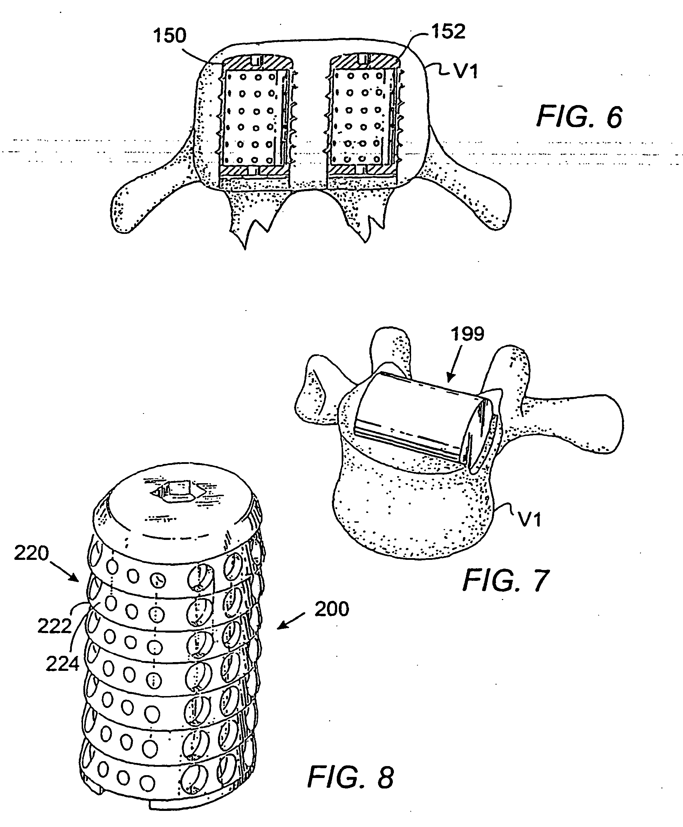

Referring to Figure 6, sectional view along lines 6--6 of Figure 5 illustrating the area of contact between the two implants 150 and 152 inserted from the anterior aspect of the spine and the vertebra V₁ is shown. As can be seen from Figure 6, the surface area of the two spinal implants 150 and 152 in contact with the vertebra V₁ is substantially less than that of a single translateral spinal fusion implant 100 that is inserted across the transverse width W of the vertebra V₁. As a result, a more stable construct is achieved with the translateral spinal fusion implant 100 of the present invention than was previously possible with implants that are inserted from either the front or the back of the patient promoting from stability of the fusion construction.

In the preferred embodiment, the spinal fusion implant 100 of the present invention has an overall length in the range of 35 mm to 50 mm, with 38-44 mm being preferred, and a maximum diameter in the range of 22 mm to 30 mm, with 24-26 mm being preferred when inserted in the lumbar spine.

Referring to Figure 7, an anterior perspective view of a single vertebra V₁ and an alternative embodiment of the translateral spinal fusion implant of the present invention, generally referred to by the numeral 199, is shown. The spinal fusion implant 199 is a dowel inserted into a hole drilled across a disc space and into the vertebra V₁ along the transverse width of the vertebra V₁. The spinal fusion implant 199 has the same dimensions as the spinal fusion implant 100 described above. The spinal fusion implant 199 can be made of any material suitable for human implantation may comprise fusion promoting and/or bioactive material to actively participate in the spinal fusion process. The implant 199 can be made of a porous, and/or mesh-like, and/or cancellous material, or any other material suitable for the described purpose.

Referring to Figure 8, an alternative embodiment of the translateral spinal fusion implant of the present invention, is shown and generally referred to by the numeral 200. The spinal fusion implant 200 has a substantially cylindrical configuration having a thin outer wall 212 surrounding an internal chamber 214. The exterior of the spinal fusion implant 200 comprises surface roughenings that provide a surface suitable for engaging the bone of the vertebrae to stabilize the spinal fusion implant 200 across the disc space and into the adjacent vertebrae once surgically implanted. The surface roughenings comprise a plurality of ratchetings 220 along the circumference of the spinal fusion implant 200. Each of the plurality of ratchetings 220 has a bone engaging edge 222 and an angled segment 224.

The spinal fusion implant 200 is implanted into a cylindrical bore derived across the disc space and into two adjacent vertebrae. The spinal fusion implant 200 may be pushed into the cylindrical bore across the disc space by direct, linear advancement since it requires no thread to pull it forward through the spine. As no torque is required to advance the spinal fusion implant 200 there is no minimum requisite height of the surface roughenings.

The ratchetings 220 may face in one direction, the direction in which the spinal fusion implant 200 is inserted, and function to prevent the spinal fusion implant 200 from backing out of the disc space in a direction opposite to the direction of insertion once inserted between the two adjacent vertebrae. The ratchetings 220 urge the spinal fusion implant 200 forward against the unremoved bone of the vertebrae. Since implants generally want to back out along the same path in which they are inserted, the ratchetings 220 tend to urge the spinal fusion implant 200 forward against the solid unremoved bone at the end of the cylindrical bone, further resisting dislodgement and controlling motion resulting in an exceedingly stable implantation.

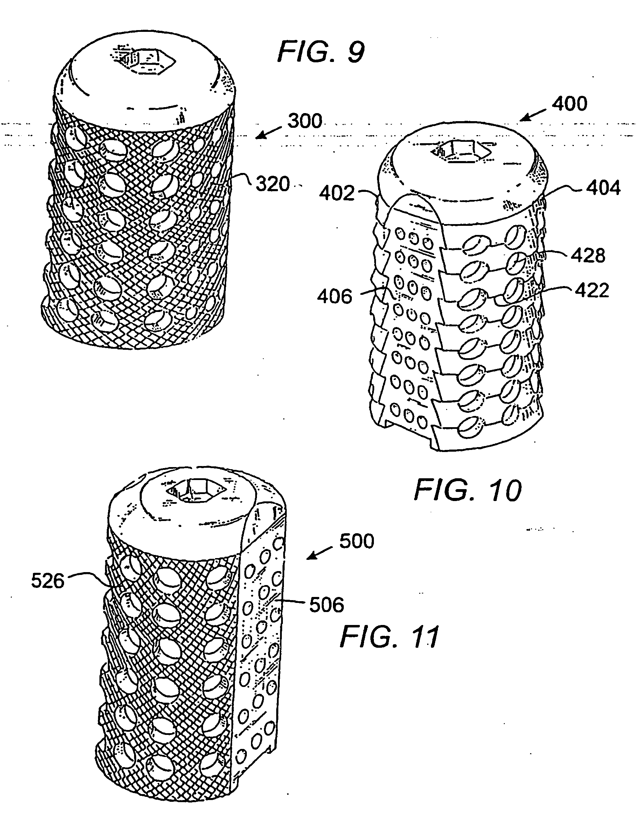

The spinal fusion implant 200 has an engagement means at one end for engaging a driver instrument for intimately engaging and binding the implant 200 and the driver instrument together. Once affixed to the implant driver instrument, the spinal fusion implant 200 may be then introduced through a hollow cylindrical tube and driven into the cylindrical hole that has been drilled across the disc space. The implant driver instrument may then be impacted by a mallet, or similar device, to linearly advance the spinal fusion implant 200 across the disc space. Once the spinal fusion implant 200 is inserted across the disc space, the ratchetings 220, engage the bone of the vertebrae and the implant driver instrument is detached from the spinal fusion implant 200.
Referring to Figure 9, an alternative embodiment of the spinal fusion implant of the present invention generally referred to by the numeral 300 is shown. The spinal fusion implant 300 has a substantially cylindrical configuration having surface roughenings for stabilizing the implant 300 within the intervertebral space D. The surface roughenings comprise a surface knurling 320 such as, but not limited to, the diamond-shaped bone engaging pattern shown in Figure 9. The spinal fusion implant 300 may have surface knurling 320 throughout the entire external surface of the spinal fusion implant 300, throughout only a portion of the external surface, or any combination thereof, without departing from the scope of the present invention. In those circumstances where there is no undrilled bone in the disc space forward of the spinal fusion implant 300 to resist further forward advancement of the implant, surface knurling 320 is preferred as it produces an exceedingly high interference fit with the bone of the vertebrae and resists motion equally in all directions and without the tendency to urge itself forward.

Referring to Figure 10, an alternative embodiment of the spinal fusion implant of the present invention is shown and is generally referred to by the numeral 400. The spinal fusion implant 400 has a similar configuration to that of the spinal fusion implant 200, except that it comprises a partially cylindrical member having arcuate portions 402 and 404 which are arcs of the same circle with portions of its outer wall that are flattened so as to present a first flat side 406. Alternatively, the implant 400 may have a second flat side that is diametrically opposite to the first flat side 406. The spinal fusion implant 400 is substantially the same as the spinal fusion implant 200, except that the openings 428 are positioned on the ratcheting 420 such that the openings 428 are positioned between the bone engaging edges 422 and are not bisected by the bone engaging edges 422.

Referring to Figure 11, an alternative embodiment of the spinal fusion implant of the present invention is shown and generally referred to by the numeral 500. The spinal fusion implant 500 is substantially identical to the spinal fusion implant 400 described above except that in place of ratchetings 420, it has surface knurling 520. The surface knurling 520 assists in the retaining of the spinal fusion implant 500 once it is inserted across the disc space between two adjacent vertebrae. It is recognized that the surface knurling 520 of the implant 500 may be combined with any of a number of other surface roughenings such as, but not limited to, ratchetings to assist in retaining the spinal fusion implant 500 across the disc space.

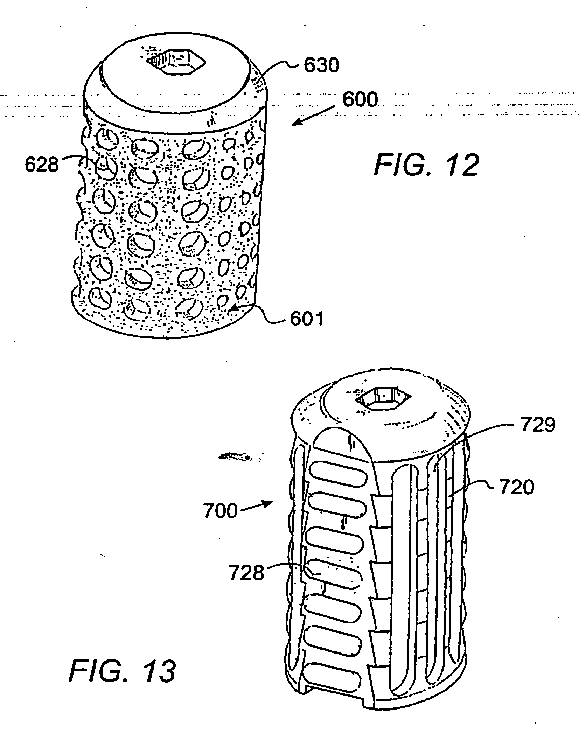

Referring to Figure 12, an alternative embodiment of the spinal fusion implant of the present invention generally referred to by the numeral 600 is shown. The spinal fusion implant 600 has the same structure as the spinal fusion implant 300 described above but instead of knurling 320 has a different surface roughening. The spinal fusion implant 600 has a surface roughening comprising of a blasted external surface 601 which may be stippled to provide an engagement surface for the vertebrae when inserted across the disc space. The spinal fusion implant has a plurality of openings 628, a removable cap 630 for accessing an internal chamber.

Referring to Figure 13, an alternative embodiment of the spinal fusion implant of the present invention generally referred to by the numeral 700 is shown. The spinal fusion implant 700 is similar to spinal fusion implant 400 described above except that it has openings in the form of horizontal slots 728 on the flat side 706 and vertical slots 729 on the cylindrical portion of the spinal fusion implant 700. The spinal fusion implant 700 has ratchetings 720 for engaging the bone of the vertebrae similar to the ratchetings 220 described above.

It is appreciated that the spinal fusion implants of the present invention may include any and all surface roughening configurations that either increase the surface area or interference fit of the implant and the vertebrae. It is appreciated that the ratchetings described above for the various embodiments of the spinal fusion implants of the present invention may also comprise a knurled or other surface roughenings in combination with the ratchetings to further enhance the retention of the spinal fusion implant across the disc space once inserted.

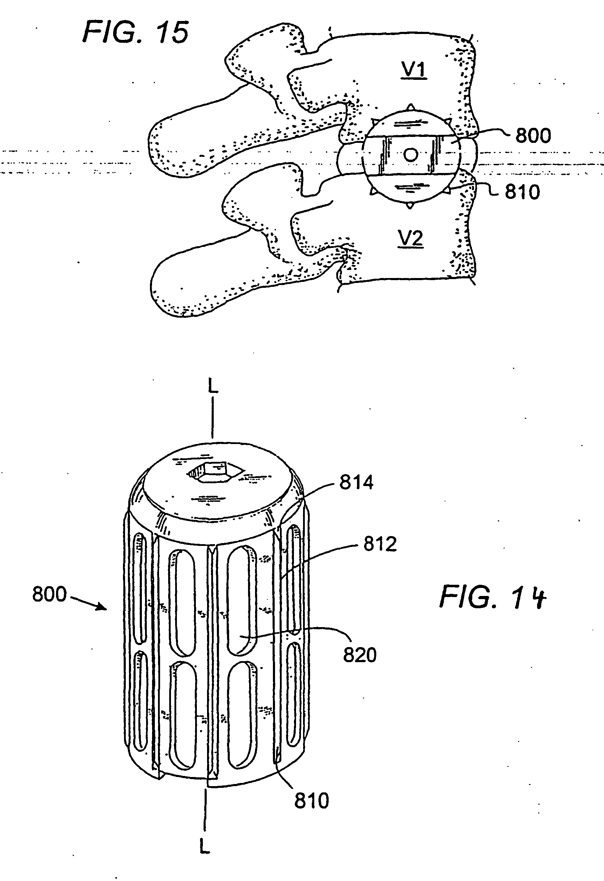

Referring to Figure 14, an alternative embodiment of the spinal fusion implant of the present invention is shown and generally referred to by the numeral 800. The spinal fusion implant 800 is similar in configuration to the spinal fusion implant 100 discussed above. However, instead of an external thread, the spinal fusion implant 800 has a plurality of longitudinal splines 810 along its external surface. The splines 810 are parallel to the central longitudinal axis L of the implant 800 in the direction of insertion of the implant 800. The splines 810 have a sharp edge 812 and a sharpened leading end 814 to facilitate insertion of the spinal fusion implant 800 into the adjacent vertebrae. Located between the splines 812 are a plurality of slots 820 that allow bone growth into the implant and into the internal chamber of the implant 800 during spinal fusion.

Referring to Figure 15, the spinal fusion implant 800 is shown inserted from the lateral aspect of the spine into a bore created across the disc space D and into the adjacent vertebrae V₁ and V₂ along the transverse width of the vertebrae V₁ and V₂. The spinal fusion implant 800 is pushed into place by linear advancement such that the splines 810 engage a portion of each of the adjacent vertebrae V₁ and V₂. The splines 810 function to engage the vertebrae V₁ and V₂ and stabilize the spinal fusion implant 800 once implanted. The splines 810 are oriented longitudinally with respect to the spinal fusion implant 800 to prevent any dislodgement of the spinal fusion implant 800 from between the vertebrae V₁ and V₂ as result of anterior to posterior motion of the spine. It is appreciated that the number of splines 810 and the configuration of the splines 810 can vary depending on the size of the spinal fusion implant 800 being implanted.

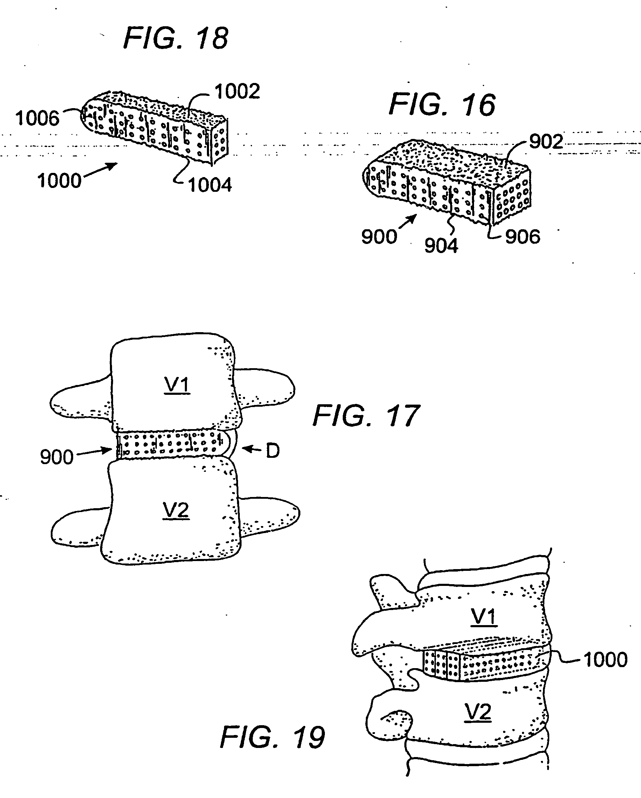

Referring to Figure 16, a spinal fusion implant is shown and generally referred to by the numeral 900. The spinal fusion implant 900 differs from the implants described above in that it is inserted in the disc space D between the adjacent vertebrae of the spine and not into a cylindrical bore created across the disc space. Therefore, the spinal fusion implant 900 does not require the removal of any portion of bone from the adjacent vertebrae as the spinal fusion implant 900 fits within the natural disc space between the adjacent vertebrae. However, the removal of at least a portion of the disc material present between the adjacent vertebrae is required for proper insertion.

The spinal fusion implant 900 comprises a rectangular block 901 having a top surface 902 and a bottom surface 904 for engaging the adjacent vertebrae and may be flat or may conform at least in part. The top and bottom surfaces 902 and 904 may comprise any of the surface roughenings described herein for engaging the bone of the adjacent vertebrae to promote firm stability. The spinal fusion implant 900 may be solid or hollow at least in part and have a plurality of openings 906 to allow bone ingrowth. The openings 906 may be present on all surfaces of the implant 900 and may either pass through the entire implant 900, or may be closed bottom wells for holding fusion promoting materials.

Referring to Figure 17, the spinal fusion implant 900 is shown implanted from the lateral aspect of the spine in the disc space D between two adjacent vertebrae V, V₁ and V₂ along the transverse width of the adjacent vertebrae V₁ and V₂. The spinal fusion implant 900 has a height that is substantially equal to the height of the disc space D, a length that is greater than one half the transverse width W of the vertebrae and a width that approximates the depth of the vertebrae.

The spinal fusion implant 900 has a height in the range of 8 mm to 16 mm, with the preferred height being 10-12 mm; a width in the range of 24 mm to 32 mm, with the preferred width being 26 mm; and a length in the range of 32 mm to 50 mm, with 42 mm being the preferred length.

Referring to Figure 18, a spinal fusion implant is shown and generally referred to by the numeral 1000. The spinal fusion implant 1000 is similar to the spinal fusion implant 900, but has a narrower width such that more than one spinal fusion implant 1000 may be combined in a modular fashion for insertion within the disc space D between the adjacent vertebrae.

Referring to Figure 19, a plurality of spinal fusion implants 1000 are shown combined in a modular fashion inserted in the disc space D from the lateral aspect of the spine and along the transverse width of the vertebrae V₁ and V₂.

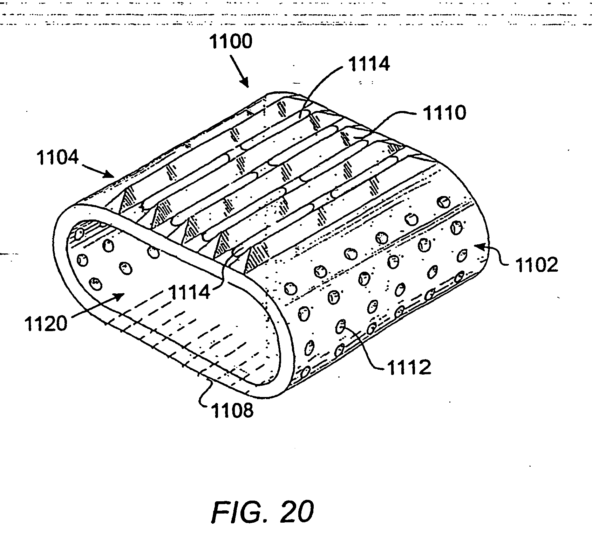

Referring to Figure 20, a spinal fusion implant is shown and generally referred to by the numeral 1100. The spinal fusion implant 1100 is inserted into the disc space between two adjacent vertebrae from the lateral aspect of the spine and along the transverse width of the vertebrae. The implant 1100 is dimensioned to replace the natural disc material present between two adjacent vertebrae. The implant 1100 has a generally rectangular body with curved sides 1102 and 1104. The top and bottom surfaces 1106 and 1108 have a plurality of splines 1110 similar in structure and function as the splines 810 described above. As the implant 1100 is inserted in the disc space, the splines 1110 engage the bone of the adjacent vertebrae.

The implant 1100 is shown as being hollow with openings 1112 and slots 1114 in the outer surface of the implant 1100 permitting bone ingrowth into the interior of the implant 1100. However, it is appreciated that the implant 1100 may be solid and may have channels or wells in place of opening 1112 to permit bone ingrowth and incorporation of the implant 1100 into the spinal fusion mass. The interior of the implant 1000 may be accessed through the aperture 1120 which may be closed with a snap fit cover.

While the present invention has been described in detail with regards to the preferred embodiment, it is appreciated that other variations of the present invention may be devised which do not depart from the inventive scope of the claims.

## Claims

1. A translateral spinal fusion implant for insertion from the lateral aspect of a human spine from a position anterior to the transverse process of the spine and into the disc space between two adjacent vertebral bodies, said implant having a length that is substantially greater than the depth of the adjacent vertebrae, said implant having a mid-longitudinal axis and opposed arcuate portions for engaging each of the adjacent vertebral bodies, said implant having a maximum height between said arcuate portions greater than 24 mm, said implant having a maximum height between said arcuate portions no greater than 30 mm, said implant having an outer surface with at least one opening passing through said implant, said at least one opening being capable of permitting bone growth from adjacent vertebral body to adjacent vertebral body through said implant.

2. The spinal fusion implant of claim 1, wherein said implant is made of a bone ingrowth inducing material.

3. The spinal fusion implant of any one of claims 1-2, further comprising surface roughenings for engaging the adjacent vertebral bodies and for maintaining said implant in place, said surface roughenings being present on at least a portion of the exterior of said implant.

4. The spinal fusion implant of claim 3, wherein said surface roughenings include at least one of a plurality of splines, ratchetings, and knurling.

5. The spinal fusion implant of any one of claims 1-2, further comprising an external thread for engaging the vertebral bodies.

6. The spinal fusion implant of any one of claims 1-5, wherein said implant has a plurality of openings capable of retaining fusion promoting material.

7. The spinal fusion implant of any one of claims 1-6, wherein said implant is modular.

8. The spinal fusion implant of any one of claims 1-6, wherein said implant comprises a plurality of modular members, each of said modular members having a length that is substantially greater than one half the transverse width of the adjacent vertebral bodies, and a width substantially less than the depth of the adjacent vertebral bodies.

9. The spinal fusion implant of claim 8, wherein each of said modular members comprises upper and lower walls, and side walls, said upper and lower walls forming a support structure including at least a portion of the interior surface of said upper and lower walls for bearing against the adjacent vertebral bodies.

10. The spinal fusion implant of any one of claims 1-9, wherein said implant has a width that is at least as great as the height of said implant.

11. The spinal fusion implant of any one of claims 1-9, wherein said implant has a width greater than the height of said implant.

12. The spinal fusion implant of any one of claims 1-9, wherein said implant has a width approximating the depth of the adjacent vertebral bodies.

13. The spinal fusion implant of any one of claims 1-6, in combination with a second spinal implant.

14. The spinal fusion implant of any one of claims 1-6, wherein said implant has a length that is greater than one half the transverse width of the adjacent vertebral bodies, and a height at least sufficient to contact each of the adjacent vertebral bodies and for restoring the height of the disc space.

15. The spinal fusion implant of any one of claims 1-14 for use in the lumbar spine, wherein said implant has a length in the range of approximately 35 mm to 50 mm.

16. The spinal fusion implant of any one of claims 1-14 for use in the thoracic spine, wherein said implant has a length in the range of approximately 12 mm to 30 mm.

17. The spinal fusion implant of any one of claims 1-16, wherein said opposed arcuate portions form a substantially cylindrical configuration.

18. The spinal fusion implant of any one of claims 1-17, wherein said implant is one of made of and coated with an osteogenic material.

19. The spinal fusion implant of claim 18, wherein said osteogenic material includes at least one of hydroxyapatite and hydroxyapatite tricalcium phosphate.

20. The spinal fusion implant of any one of claims 1-17, further comprising a fusion promoting material.

21. The spinal fusion implant of claim 20, wherein said fusion promoting material includes at least one of bone, bone morphogenetic protein, hydroxyapatite, and hydroxyapatite tricalcium phosphate.

## Patentansprüche

1. Translaterales Wirbelsäulenfusionsimplantat zum Einsetzen von der Seite einer menschlichen Wirbelsäule her aus einer Position anterior bzw. vorderseitig zum Querfortsatz bzw. Querverlauf der Wirbelsäule in den Bandscheibenzwischenraum zwischen zwei benachbarten Wirbelkörpern hinein, wobei das Implantat eine Länge aufweist, die im Wesentlichen größer als die Tiefe der benachbarten Wirbelkörper ist, das Implantat eine Mittenlängsachse und gegenüberliegende gekrümmte Abschnitte für den Eingriff mit jedem der benachbarten Wirbelkörper aufweist, das Implantat eine maximale Höhe zwischen den gekrümmten Abschnitten von mehr als 24 mm aufweist, das Implantat eine maximale Höhe zwischen den gekrümmten Abschnitten von nicht mehr als 30 mm aufweist, das Implantat eine äußere Oberfläche mit wenigstens einer durch das Implantat verlaufenden Öffnung aufweist, und die wenigstens eine Öffnung Knochenwachstum von einem benachbarten Wirbelkörper zu einem benachbarten Wirbelkörper durch das Implantat ermöglicht.

2. Wirbelsäulenfusionsimplantat nach Anspruch 1, wobei das Implantat aus einem knocheneinwuchsinduzierenden Material besteht.

3. Wirbelsäulenfusionsimplantat nach einem der Ansprüche 1 bis 2, des Weiteren umfassend Oberflächenaufrauungen für den Eingriff mit den benachbarten Wirbelkörpern und zum Festhalten des Implantates, wobei die Oberflächenaufrauungen an wenigstens einem Abschnitt des Äußeren des Implantates vorhanden sind.

4. Wirbelsäulenfusionsimplantat nach Anspruch 3, wobei die Oberflächenaufrauungen aus einer Mehrzahl von Rillen, Sperren und Riffelungen wenigstens eines enthalten.

5. Wirbelsäulenfusionsimplantat nach einem der Ansprüche 1 bis 2, des Weiteren umfassend ein Außengewinde für den Eingriff mit den Wirbelkörpern.

6. Wirbelsäulenfusionsimplantat nach einem der Ansprüche 1 bis 5, wobei das Implantat eine Mehrzahl von Öffnungen aufweist, die ein fusionsförderndes Material halten können.

7. Wirbelsäulenfusionsimplantat nach einem der Ansprüche 1 bis 6, wobei das Implantat modular ist.

8. Wirbelsäulenfusionsimplantat nach einem der Ansprüche 1 bis 6, wobei das Implantat eine Mehrzahl von modularen Gliedern umfasst und jedes der modularen Glieder eine Länge, die im Wesentlichen größer als eine Hälfte der Querbreite der benachbarten Wirbelkörper ist, und eine Breite, die im Wesentlichen kleiner als die Breite der benachbarten Wirbelkörper ist, aufweist.

9. Wirbelsäulenfusionsimplantat nach Anspruch 8, wobei jedes der modularen Glieder obere und untere Wände sowie Seitenwände umfasst und die oberen und unteren Wände eine Trägerstruktur bilden, die wenigstens einen Abschnitt der inneren Oberfläche der oberen und unteren Wände zum Abstützen an den benachbarten Wirbelkörpern enthält.

10. Wirbelsäulenfusionsimplantat nach einem der Ansprüche 1 bis 9, wobei das Implantat eine Breite aufweist, die wenigstens so groß wie die Höhe des Implantates ist.

11. Wirbelsäulenfusionsimplantat nach einem der Ansprüche 1 bis 9, wobei das Implantat eine Breite aufweist, die größer als die Höhe des Implantates ist.

12. Wirbelsäulenfusionsimplantat nach einem der Ansprüche 1 bis 9, wobei das Implantat eine Breite aufweist, die annähernd gleich der Tiefe der benachbarten Wirbelkörper ist.

13. Wirbelsäulenfusionsimplantat nach einem der Ansprüche 1 bis 6 in Kombination mit einem zweiten Wirbesäulenimplantat.

14. Wirbelsäulenfusionsimplantat nach einem der Ansprüche 1 bis 6, wobei das Implantat eine Länge, die größer als eine Hälfte der Querbreite der benachbarten Wirbelkörper ist, und eine Höhe, die wenigstens ausreichend ist, um jeden der benachbarten Wirbelkörper zu kontaktieren und die Höhe des Bandscheibenzwischenraumes wiederherzustellen, aufweist.

15. Wirbelsäulenfusionsimplantat nach einem der Ansprüche 1 bis 14 zur Verwendung in der Lendenwirbelsäule, wobei das Implantat eine Länge in dem Bereich von annähernd 35 mm bis 50 mm aufweist.

16. Wirbelsäulenfusionsimplantat nach einem der Ansprüche 1 bis 14 zur Verwendung in der Brustwirbelsäule, wobei das Implantat eine Länge in dem Bereich von annähernd 12 mm bis 30 mm aufweist.

17. Wirbelsäulenfusionsimplantat nach einem der Ansprüche 1 bis 16, wobei die gegenüberliegenden gekrümmten Abschnitte einen im Wesentlichen zylindrischen Aufbau bilden.

18. Wirbelsäulenfusionsimplantat nach einem der Ansprüche 1 bis 17, wobei das Implantat entweder aus einem osteogenen Material besteht oder mit einem solchen beschichtet ist.

19. Wirbelsäulenfusionsimplantat nach Anspruch 18, wobei das osteogene Material von Hydroxyapatit und Hydroxyapatit-Tricalciumphosphat wenigstens eines enthält.

20. Wirbelsäulenfusionsimplantat nach einem der Ansprüche 1 bis 17, des Weiteren umfassend ein fusionsförderndes Material.

21. Wirbelsäulenfusionsimplantat nach Anspruch 20, wobei das fusionsfördernde Material von Knochen, knochenmorphogenetischem Protein, Hydroxyapatit und Hydroxyapatit-Tricalciumphosphat wenigstens eines enthält.

## Revendications

1. Implant translatéral de fusion spinale pour insertion depuis le faciès latéral d'une colonne vertébrale humaine à partir d'une position antérieure à l'apophyse transverse de la colonne vertébrale et dans l'espace discal entre deux corps vertébraux adjacents, ledit implant ayant une longueur qui est substantiellement supérieure à la profondeur des vertèbres adjacentes, ledit implant ayant un axe longitudinal moyen et des parties arquées opposées pour engager chacun des corps vertébraux adjacents, ledit implant ayant une hauteur maximale entre lesdites parties arquées supérieure à 24 mm, ledit implant ayant une hauteur maximale entre lesdites parties arquées non supérieure à 30 mm, ledit implant ayant une surface externe comportant au moins une ouverture passant au travers dudit implant, ladite au moins une ouverture étant capable de permettre la croissance osseuse d'un corps vertébral adjacent à un corps vertébral adjacent au travers dudit implant.

2. Implant de fusion spinale selon la revendication 1, dans lequel ledit implant est constitué d'un matériau induisant la croissance osseuse.

3. Implant de fusion spinale selon l'une quelconque des revendications 1 et 2, comprenant en outre des rugosités de surface pour engager les corps vertébraux adjacents et pour maintenir ledit implant en place, lesdites rugosités de surface étant présentes sur au moins une partie de l'extérieur dudit implant.

4. Implant de fusion spinale selon la revendication 3, dans lequel lesdites rugosités de surface comprennent au moins l'un parmi une pluralité de languettes, de cliquets et de moletages.

5. Implant de fusion spinale selon l'une quelconque des revendications 1 et 2, comprenant en outre un filetage externe pour engager les corps vertébraux.

6. Implant de fusion spinale selon l'une quelconque des revendications 1 à 5, dans lequel ledit implant a une pluralité d'ouvertures capable de retenir un matériau favorisant la fusion.

7. Implant de fusion spinale selon l'une quelconque des revendications 1 à 6, dans lequel ledit implant est modulaire.

8. Implant de fusion spinale selon l'une quelconque des revendications 1 à 6, dans lequel ledit implant comprend une pluralité d'éléments modulaires, chacun desdits éléments modulaires ayant une longueur qui est substantiellement supérieure à la moitié de la largeur transversale des corps vertébraux adjacents, et une largeur substantiellement inférieure à la profondeur des corps vertébraux adjacents.

9. Implant de fusion spinale selon la revendication 8, dans lequel chacun desdits éléments modulaires comprend des parois supérieure et inférieure, et des parois latérale, lesdites parois supérieure et inférieure formant une structure de support comprenant au moins une partie de la surface intérieure desdites parois supérieure et inférieure destinée à porter contre lesdits corps vertébraux adjacents.

10. Implant de fusion spinale selon l'une quelconque des revendications 1 à 9, dans lequel ledit implant a une largeur qui est au moins aussi grande que la hauteur dudit implant.

11. Implant de fusion spinale selon l'une quelconque des revendications 1 à 9, dans lequel ledit implant a une largeur supérieure à la hauteur dudit implant.

12. Implant de fusion spinale selon l'une quelconque des revendications 1 à 9, dans lequel ledit implant a une largeur s'approchant de la profondeur des corps vertébraux adjacents.

13. Implant de fusion spinale selon l'une quelconque des revendications 1 à 6, en combinaison avec un second implant spinal.

14. Implant de fusion spinale selon l'une quelconque des revendications 1 à 6, dans lequel ledit implant a une longueur qui est supérieure à la moitié de la largeur transversale des corps vertébraux adjacents et une hauteur au moins suffisante pour entrer en contact avec chacun des corps vertébraux adjacents et pour restaurer la hauteur de l'espace discal.

15. Implant de fusion spinale selon l'une quelconque des revendications 1 à 14 pour utilisation dans la colonne lombaire, dans lequel ledit implant a une longueur dans l'intervalle d'environ 35 mm à 50 mm.

16. Implant de fusion spinale selon l'une quelconque des revendications 1 à 14, pour utilisation dans la colonne thoracique, dans lequel ledit implant a une longueur dans l'intervalle d'environ 12 mm à 30 mm.

17. Implant de fusion spinale selon l'une quelconque des revendications 1 à 16, dans lequel lesdites parties arquées opposées forment une configuration substantiellement cylindrique.

18. Implant de fusion spinale selon l'une quelconque des revendications 1 à 17, dans lequel ledit implant est constitué et revêtu d'un matériau ostéogène.

19. Implant de fusion spinale selon la revendication 18, dans lequel ledit matériau ostéogène comprend au moins un matériau parmi l'hydroxyapatite et l'hydroxyapatite triphosphate de calcium.

20. Implant de fusion spinale selon l'une quelconque des revendications 1 à 17, comprenant en outre un matériau favorisant la fusion.

21. Implant de fusion spinale selon la revendication 20, dans lequel ledit matériau favorisant la fusion comprend au moins un matériau parmi l'os, une protéine morphogénétique osseuse, l'hydroxyapatite et l'hydroxyapatite triphosphate de calcium.
